# FASCICULE DE BREVET EUROPEEN

(11) **EP 2 501 335 B1**
(45) Date de publication et mention de la délivrance du brevet: **23.10.2013**
(21) Numéro de dépôt: 10792988.7
(22) Date de dépôt: 10.11.2010
(51) Int. Cl.: A61F 2/12

(54) **IMPLANT DESTINÉ À LA RECONSTRUCTION ANATOMIQUE OU L'AUGMENTATION VOLUMÉTRIQUE**
IMPLANTAT FÜR EINE ANATOMISCHE REKONSTRUKTION ODER ANATOMISCHE VOLUMENVERGRÖSSERUNG
IMPLANT FOR ANATOMICAL RECONSTRUCTION OR VOLUME AUGMENTATION

(30) Priorité: 20.11.2009 FR 0958210
(43) Date de publication de la demande: 26.09.2012
(73) Titulaire: Brinon, Thierry, 83140 Six-Fours-les-Plages (FR)
(72) Inventeur: Brinon, Thierry, 83140 Six-Fours-les-Plages (FR)
(74) Mandataire: Novagraaf Technologies
(86) Numéro de dépôt international: PCT/FR2010/052410
(87) Numéro de publication internationale: WO 2011/061433

(56) Documents cités:
- US-A- 4 731 081
- US-A- 5 961 552
- US-A1- 2001 010 024

## Description

La présente invention concerne un implant destiné à la reconstruction anatomique ou l'augmentation volumétrique d'une partie molle d'un corps vivant.

L'application principale de l'invention est le domaine des implants ou prothèses mammaires adaptés pour être implantés chirurgicalement sous la peau d'une personne, en remplacement ou en complément de la glande mammaire pour respectivement une reconstruction anatomique et réparatrice après une mastectomie ou ablation de cette glande, ou une augmentation volumétrique pour des raisons esthétiques.

D'autres applications pour d'autres zones corporelles sont également possibles avec des implants suivant la présente invention, mais dans la description suivante on citera et prendra l'exemple le plus connu des implants mammaires.

On connaît de telles prothèses qui comportent une enveloppe en élastomère, de préférence en silicone, et du gel de silicone ou du sérum physiologique qui remplit ladite enveloppe : leurs formes sont soit rondes, soit anatomiques symétriques, soit anatomiques asymétriques suivant l'effet recherché.

De nombreuses demandes de brevet ont été déposées pour couvrir diverses techniques de fabrication, telle que la demande de brevet FR 2 735 354 déposée le 13 juin 1995 par le LABORATOIRE PEROUSE IMPLANT qui décrit une prothèse mammaire comportant une pluralité de poches remplies de sérum physiologique et remplissant l'enveloppe externe pour réduire la mobilité de ce fluide et obtenir des propriétés mécaniques proches de celles d'une glande mammaire naturelle.

Quelle que soit la forme ou la technique de fabrication de tels implants ou prothèses, on a constaté une première complication, à présent très connue, post opératoire après mise en place de l'implant : la contracture capsulaire. En effet, la réaction physiologique, normale et constante de l'organisme humain en présence d'un corps étranger, est de l'isoler des tissus environnants en constituant une membrane hermétique qui va entourer l'implant et qu'on appelle «capsule périprothétique». Normalement, cette membrane est fine, souple et imperceptible, mais il arrive que la réaction s'amplifie et que la capsule s'épaississe, devienne fibreuse et se rétracte en comprimant l'implant, prenant alors le nom de «coque». Selon l'intensité du phénomène, il peut en résulter : un simple raffermissement du sein, une constriction parfois gênante, voire une déformation visible avec globulisation de la prothèse aboutissant à l'extrême à une sphère dure, douloureuse, plus ou moins excentrée.

Cette fibrose rétractile, appelée aussi contracture capsulaire, est parfois secondaire à un hématome ou une infection, mais la plupart du temps sa survenue reste imprévisible, résultant de réactions organiques aléatoires.

De gros progrès ont été réalisés ces dernières années en matière, d'une part de techniques chirurgicales, et d'autre part et surtout de conception et de constitution des implants, aboutissant à une diminution très sensible du taux de coques (ou contractures capsulaires) et de leur intensité, et ceci grâce aux implants à surface externe texturée.

La surface externe rugueuse de l'implant est généralement obtenue en utilisant deux types de procédé de fabrication.

Le premier consiste à projeter sur la dernière couche trempée dans la dispersion de silicone des cristaux solubles dans l'eau, généralement du sucre ou du sel. Après catalyse de l'enveloppe (cuisson), les enveloppes sont plongées dans de l'eau pour dissoudre les cristaux. Après dissolution, l'empreinte laissée sur l'enveloppe constitue la surface rugueuse de celle-ci. La granulométrie, la forme et la force de projection des cristaux définissent l'état de surface final de l'enveloppe (rugosité, profondeur et densité des pores). On pourrait citer pour illustrer ce premier type de procédé le brevet FR 2 637 537 du 11 octobre 1989 de la société US CUI CORPORATION, ainsi que la demande de brevet WO 2009/061672 du 31 octobre 2008 de la société ALLERGAN ou encore le brevet US 5 674 285 déposé le 12 décembre 1995 par la société MEDICAL PRODUCTS DEVELOPMENT.

L'autre procédé connu de fabrication consiste à réaliser la surface rugueuse sur le moule de l'enveloppe. L'état de surface du moule peut être obtenu soit par grenaillage avec un abrasif calibré, soit par usinage, soit par moulage, soit par tout autre technique permettant de modifier l'état de surface du moule. Dans ce cas, c'est la surface de l'enveloppe en contact avec le moule qui devient rugueuse et, après démoulage, l'enveloppe est alors retournée pour obtenir la surface rugueuse externe de l'implant. On peut citer pour illustrer ce deuxième type de procédé le brevet EP 1 847 369 du 19 avril 2007 de la société CEREPLAS.

Ainsi, les implants mammaires connus à ce jour peuvent être constitués d'une enveloppe dont la surface externe est lisse ou texturée.

Une deuxième complication post opératoire est celle de la diffusion d'huile au travers de l'enveloppe et pouvant engendrer la formation de siliconome : en effet, le gel de silicone, qui est le produit souvent utilisé pour le remplissage de l'enveloppe afin de lui donner son volume et apporter à l'implant les propriétés mécaniques souhaitées, est obtenu grâce à un mélange majoritairement constitué d'huile de silicone et d'un catalyseur. La consistance du gel est obtenue après réticulation (cuisson). Une fois réticulé, en vieillissant à l'intérieur de l'enveloppe, le gel libère en surface des chaînes moléculaires dites courtes se traduisant par une remontée d'huile à la surface du gel puis, au travers de l'enveloppe de l'implant. Ce phénomène, appelé la transsudation, est bien connu de l'homme de l'art.

Les fabricants de silicone et d'implants mammaires ont développé des solutions pour rendre moins perméables les enveloppes d'implants et ainsi, limiter la migration des chaînes courtes vers l'extérieur de l'implant : on peut citer dans ce cadre la demande de brevet FR 2 498 446 de la société INSERM du 26 janvier 1981 qui décrit un implant à surface externe hydrophile et qui, du reste, initialement a été mise au point pour pallier la première complication citée précédemment de contractures capsulaires.

Une autre conséquence de la remontée de l'huile à la surface du gel mais qui est moins décrite bien que néanmoins connue, est la désolidarisation du gel de l'enveloppe. Ceci peut entraîner la déformation de l'implant et l'apparition de plicatures de l'enveloppe. Ceci peut se traduire par une dégradation du résultat esthétique de l'implantation, mais également par une dégradation prématurée de l'enveloppe par cisaillement. Cette dégradation pouvant aller jusqu'à la rupture de l'enveloppe qui est alors une troisième complication post opératoire.

La rupture des enveloppes d'implants mammaires est en fait le risque à ce jour le plus courant rencontré avec ce type d'implant, les deux complications décrites précédemment pouvant être considérées à présent maîtrisées.

En effet, même si l'on peut estimer la durée de vie d'un implant mammaire à dix voire douze ans d'implantation, nombre de cas ont montré des ruptures d'enveloppe prématurées bien avant ces échéances.

Les causes des ces ruptures sont multiples.

On peut distinguer deux catégories de ruptures d'implant :
- les ruptures indépendantes de la qualité de l'implant,
- les ruptures directement liées à la qualité de l'implant.

Parmi les causes de rupture d'enveloppe d'implant mammaire indépendantes de la qualité de l'implant, on peut citer le traumatisme lié à un choc violent, l'altération de l'enveloppe lors de l'implantation (instruments chirurgicaux, aiguille de suture) ou encore le mal-positionnement de l'implant entraînant des plis de l'enveloppe.

La qualité de conception et de fabrication de l'implant peut être également une cause de rupture prématurée. En effet, le choix des matériaux, la régularité d'épaisseur de l'enveloppe, la maîtrise du procédé de fabrication sont autant de paramètres qui peuvent impacter la résistance mécanique de l'enveloppe.

Les élastomères de silicone avec lesquels sont généralement réalisées les enveloppes des prothèses sont connus pour leur excellente tolérance par le corps humain (biocompatibilité) et leurs propriétés mécaniques particulièrement adaptées aux implants mammaires : souplesse, élasticité et mémoire de forme (rémanence). Néanmoins, la résistance à la déchirure de ces polymères demeure leur principale faiblesse pour ce type d'application.

Or, dès qu'une amorce de rupture d'enveloppe d'implant mammaire apparaît, celle-ci évolue et ce propage consécutivement aux sollicitations de l'implant (mouvements, pressions, etc.). Après quelques semaines ou mois, une rupture de quelques millimètres peut atteindre plusieurs centimètres.

Et, contrairement aux implants mammaires pré-remplis de sérum physiologique, dont la rupture d'enveloppe est immédiatement diagnosticable (l'implant se vide), une rupture d'implant pré-rempli de gel de silicone peut être qualifiée de « silencieuse ». En effet, tant que la taille de l'ouverture ne permet pas la migration du gel, la rupture est quasi indécelable, ni par palpation ni par les moyens d'imagerie tels que radiographie, échographie, IRM.

C'est lorsque que le gel a migré hors de l'enveloppe que la rupture est généralement diagnostiquée.

Certes, depuis plusieurs années, les fabricants ont mis au point des gels de silicone dits de haute cohésivité afin de limiter les risques de fragmentation du gel en cas de rupture d'enveloppe. Mais cette cohésivité est toute relative, et de toute façon, la cohésivité du gel n'exclut pas l'extraction partielle ou totale de gel hors de l'enveloppe en cas de déchirure d'enveloppe importante.

La migration du gel consécutive à une rupture d'implant est, dans tous les cas, facteur de complications (douleurs, ganglions axillaires) et impose une intervention chirurgicale (explantation).

Lors de cette intervention, après extraction de l'implant rompu, il est impératif de procéder à un curetage de la loge et des tissus voisins afin d'extraire toute trace de gel avant mise en place d'un nouvel implant. Cependant, l'extraction totale des fragments de gel étant particulièrement délicate et aléatoire, il est fréquent de voir réapparaître des siliconomes quelques mois après le remplacement de l'implant. Le document US 5 961 552 divulgue le préambule de la revendication 1.

L'objectif de la présente invention est ainsi d'éviter cette troisième complication post opératoire en proposant un implant en particulier mammaire, pré-rempli de gel de silicone, ne présentant pas de risque de déformation de l'implant due à la désolidarisation du gel de l'enveloppe et qui en cas de rupture, et ce quelle que soit la taille de la rupture, garantisse le maintien du gel dans l'enveloppe.

Cet objectif est atteint par un implant destiné à la reconstruction anatomique ou à l'augmentation volumétrique d'une partie molle d'un corps vivant, tel que présenté dans les revendications, lequel implant est constitué d'une enveloppe en matériau souple et biocompatible et rempli d'un gel, et la surface interne de l'enveloppe en contact avec le gel est, suivant l'invention, texturée suivant le sens et la définition donnés ci-après, à savoir comportant de petites zones en creux, appelées des alvéoles, qui sont ouvertes, régulières ou non, et séparées par des zones appelées aspérités dites alors en relief ; le diamètre et la profondeur des alvéoles, comme la largeur et la hauteur des aspérités, sont de quelques microns (µm) : les dimensions microscopiques des alvéoles et aspérités donnent à la surface interne de l'enveloppe un état de rugosité fine.

Dans un mode préférentiel de l'invention l'enveloppe est en élastomère et le gel est un gel de silicone.

Dans l'application principale de l'invention l'implant est un implant mammaire de forme ronde, anatomique symétrique ou asymétrique.

Le résultat est un nouvel implant dont l'état de surface rugueux de l'intérieur de l'enveloppe permet une accroche surprenamment efficace du gel à l'enveloppe, qu'on peut qualifier de liaison cohésive.

En effet, lors de différents essais dont celui de décollement de l'enveloppe avec des implants connus qui ont des surfaces internes lisses, il a été constaté pour illustrer la troisième complication post opératoire décrite précédemment qu'en maintenant l'implant suspendu par le sommet de son dôme accroché à une pince (type pince à linge) le poids de l'implant applique une traction qui tend à désolidariser l'enveloppe du gel : ainsi, en contrôlant à intervalles réguliers le décollement de l'enveloppe, les résultats ont montré que le décollement ou la désolidarisation, qu'on peut qualifier de rupture adhésive, du gel contenu dans l'implant avec l'enveloppe à surface interne lisse est constaté dès le 10^{ème} jour, et se poursuit au-delà du 30^{ème} jour.

Par contre, en prenant un implant avec enveloppe à surface interne texturée ayant suivi le même cycle complet de fabrication, stérilisation incluse, que l'implant à surface lisse ci-dessus, et avec des échantillons de même forme, de même volume et fabriqués avec le même lot de gel de remplissage et suivant les mêmes conditions de fabrication, aucun décollement n'a été constaté avec cet implant dont l'enveloppe a une surface interne texturée, et ce d'une manière surprenante, même après 100 jours de suspension. Certes, on peut considérer qu'une telle surface dispose d'une mouillabilité accrue par rapport à une surface lisse ou à des alvéoles non microscopiques, et augmente l'adhérence mécanique entre le gel et l'enveloppe, mais l'absence de décollement obtenu n'en reste pas moins surprenant.

Le nouvel implant suivant l'invention répond à l'objectif défini précédemment et les résultats d'essais décrits ci-dessus et ceux décrits ci-après en prouvent l'intérêt : la description et les figures jointes en donnent des exemples de réalisation, sachant que d'autres modes de réalisation sont cependant possibles dans le cadre de la portée de la présente invention.
La figure 1 est un exemple d'implant mammaire rond en vue perspective.
La figure 2A montre un implant suivant l'invention dont l'enveloppe a été découpée et reste solidaire du gel malgré la pression exercée sur l'implant, le gel n'étant pas extrait de l'enveloppe.
La figure 2B montre un implant connu dont l'enveloppe est à surface interne lisse et a été découpée comme pour l'implant de la figure 2A : la pression exercée sur l'implant décolle l'enveloppe du gel qui sort de la prothèse.
La figure 3A montre un essai de traction d'une éprouvette découpée sur le dôme d'un implant suivant l'invention : il n'y a pas de décollement du gel et ce dernier se fracture en restant adhérent à l'éprouvette.
La figure 3B montre l'essai de traction d'une éprouvette comme sur la figure 3A, découpée sur le dôme d'un implant connu dont l'enveloppe est lisse à l'intérieur : l'éprouvette se décolle du gel et aucune trace de celui-ci n'est visible sur l'éprouvette.

Tout implant 1 destiné à la reconstruction anatomique ou à l'augmentation volumétrique d'une partie molle d'un corps vivant, tels que les implants ou prothèses mammaires, est constitué d'une enveloppe 3 en matériau souple et biocompatible, de préférence en élastomère, et rempli d'un gel 2, de préférence de silicone.

La surface externe 4₁ de l'enveloppe 3 est soit lisse, soit texturée ou rugueuse et, suivant l'invention, la surface interne 4₂ de l'enveloppe 3 en contact avec le gel 2 est texturée, à savoir comportant des petites zones en creux, appelées des alvéoles, qui sont ouvertes, et régulières ou non, et séparées par des zones appelées aspérités dites alors en relief ; on peut considérer qu'une telle surface interne dite texturée est également rugueuse.

Pour obtenir l'effet texturé ou rugueux voulu, la profondeur et le diamètre des alvéoles de la surface interne texturée de l'enveloppe 3 sont microscopiques, comprises de préférence entre 5 et 40 µm, et la densité de ces alvéoles est de préférence comprise entre 300 alvéoles/cm² et 1800 alvéoles/cm²; de même les aspérités, qui sont de même hauteur microscopique que la profondeur des alvéoles, sont aussi de préférence de largeur microscopique comprise entre 5 et 40 µm ; ces alvéoles sont réalisées par tout procédé connu lors de la fabrication de ladite enveloppe 3.

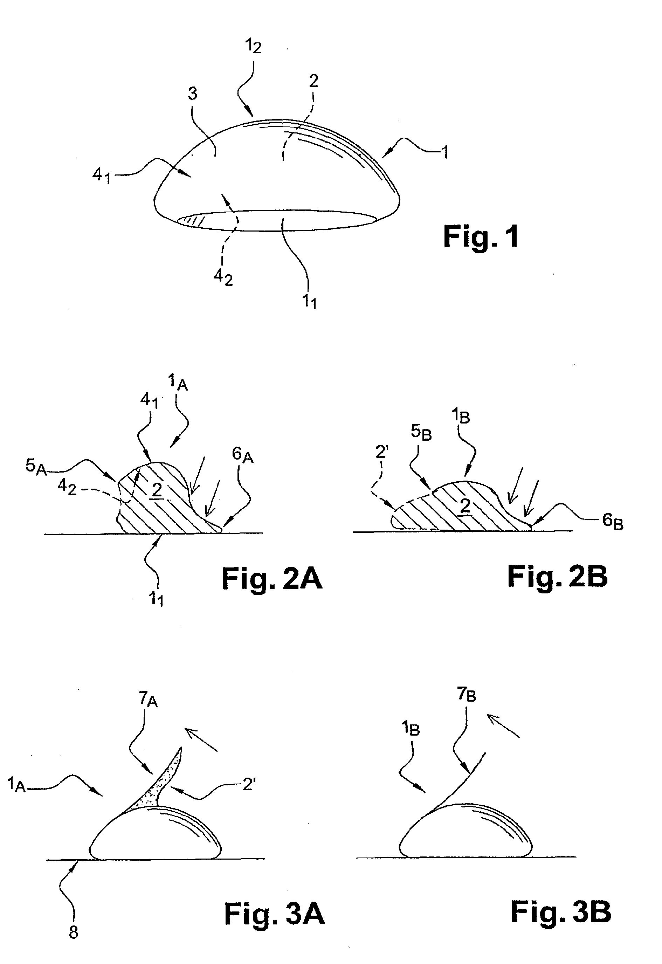

Dans le mode de réalisation de la figure 1, l'implant mammaire 1 représenté est rond avec une partie 1₂ en forme de dôme destiné à reproduire la forme anatomique du sein que l'on veut reconstituer ou dont on veut augmenter le volume, avec une base plane 1₁.

L'implant 1 mammaire peut être également de forme anatomique symétrique ou asymétrique.

Les figures 2 et 3 montrent deux essais complémentaires à celui décrit précédemment et qui confirment, d'une part que les implants suivant la présente invention répondent bien au problème posé et que, d'autre part, les implants connus à ce jour peuvent provoquer la troisième complication post opératoire décrite précédemment en cas de rupture de l'enveloppe de l'implant.

Les figures 2 représentent des essais comparatifs dits essais de migration, qui consistent à pratiquer une ouverture 5 de l'implant 1 découpée, par exemple avec un scalpel et sur une longueur correspondant à la demi-circonférence de l'implant représenté sur la figure 1 ; l'équateur étant le rayon de raccordement entre la base 1₁ et le dôme 1₂ de l'implant. Il est rappelé, comme dans la description du premier essai décrit précédemment, que les implants la représentés sur les figures 2A et 3A suivant l'invention et ceux 1b représentés sur les figures 2B et 3B correspondant aux implants connus à ce jour, sont des échantillons de même forme, de même volume et fabriqués avec le même lot de gel de remplissage et suivant les mêmes conditions de fabrication avec un cycle complet identique, stérilisation incluse.

Dans ce deuxième essai les échantillons sont ensuite maintenus suspendus par une pince accrochée à l'opposé 6a de l'ouverture 5a, respectivement 6b et 5b, et toutes les vingt-quatre heures, chaque implant subit cinq pressions successives en posant, suivant les figures 2, ces implants sur une surface plane 8 et en appuyant sur le dôme 1₂ afin de reproduire les sollicitations que peut subir une prothèse implantée dans un corps humain et dont l'enveloppe s'est rompue.

A intervalles réguliers, le décollage et le fluage du gel 2 hors de l'enveloppe 3 sont vérifiés.

De tels essais ont ainsi montré que le gel 2, enfermé dans l'enveloppe 3 à surface interne lisse des implants connus à ce jour, se désolidarise progressivement de cette enveloppe 3 et s'extrait progressivement 2' de cette enveloppe après 40 jours de suspension. Après 130 jours de suspension le gel 2 s'est totalement extrait 2' de l'enveloppe 3.

Par contre, l'implant 1a suivant la figure 2A, dont l'enveloppe 3 est à surface interne texturée selon l'invention, n'a relevé aucun décollement ni extrusion du gel 2 hors de l'enveloppe, même après 280 jours de suspension.

La série de troisièmes essais représentée sur les figures 3 consiste à mesurer avec un dynamomètre la force de traction nécessaire au décollement de l'enveloppe 2 et d'analyser la surface interne de celle-ci aux termes de l'essai. Pour cela une éprouvette rectangulaire de 100 mm/15 mm de côté, centrée et répartie symétriquement au sommet du dôme 1₂ de chaque implant 1_{A} et 1_{B}, est découpée et laissée en place.

Une des deux extrémités de l'éprouvette 7 est pincée dans la mâchoire mobile d'un dynamomètre et la base de l'implant est maintenue sur le plateau fixe 8 du dynamomètre. Les éprouvettes 7_{A} et 7_{B} sont tractées à une vitesse de 20 mm par minute jusqu'au décollement total de l'éprouvette considérée, en contact avec le gel 2.

L'essai de la figure 3B, dans lequel l'implant 1_{B} est à surface interne lisse, a montré que l'éprouvette 7_{B} se décollait avec une force inférieure à 0,5 Newton et qu'aux termes de l'essai la surface interne lisse de l'éprouvette 7_{B} ne présentait aucune trace de gel.

A l'inverse, sur la figure 3A avec un implant suivant l'invention à surface interne texturée, l'éprouvette 7_{A} s'est désolidarisée du gel 2 avec une force de 0,7 Newton et était entièrement recouverte de gel 2' aux termes de l'essai. Dans ce cas, le gel s'est fracturé.

Comme décrit précédemment, la texturation de l'enveloppe 3 peut être obtenue :
- soit par projection de cristaux solubles à la surface de l'élastomère non réticulé, puis par dissolution des cristaux après réticulation de l'élastomère,
- soit par modification de l'état de la surface du moule sur lequel est réalisée l'enveloppe en élastomère.

Dans le cas de la réalisation de l'implant mammaire objet de l'invention dont la surface externe 4₁ de l'enveloppe 3 est lisse, la texture interne 4₂ de l'enveloppe peut être obtenue grâce à l'un ou l'autre des deux procédés décrits ci-avant.

Dans le cas de la réalisation de l'implant mammaire objet de l'invention dont la surface externe 4₁ est texturée, les deux procédés sont alors nécessaires : la texture de la face interne sur le moule et la texture de la surface externe par projection de cristaux solubles ou inversement.

## Revendications

1. Implant (1) destiné à la reconstruction anatomique ou à l'augmentation volumétrique d'une partie molle d'un corps vivant, lequel est constitué d'une enveloppe (3) en matériau souple et biocompatible et remplie d'un gel (2), la surface interne (4₂) de l'enveloppe (3) en contact avec le gel étant texturée, **caractérisé en ce que** ladite surface comporte des alvéoles ouvertes de dimensions microscopiques.

2. Implant selon la revendication 1, **caractérisé en ce que** l'enveloppe (3) est en élastomère.

3. Implant selon l'une quelconque des revendications 1 ou 2, **caractérisé en ce que** le gel (2) est un gel de silicone.

4. Implant selon l'une quelconque des revendications 1 à 3, **caractérisé en ce que** la surface externe (4₁) de l'enveloppe (3) est lisse.

5. Implant selon l'une quelconque des revendications 1 à 3, **caractérisé en ce que** la surface externe (4₁) de l'enveloppe (3) est texturée.

6. Implant selon l'une quelconque des revendications 1 à 5, **caractérisé en ce que** l'implant (1) est un implant mammaire de forme ronde.

7. Implant selon l'une quelconque des revendications 1 à 5, **caractérisé en ce que** l'implant (1) est un implant mammaire de forme anatomique symétrique ou asymétrique.

8. Implant selon l'une quelconque des revendications 1 à 7, **caractérisé en ce que** la profondeur des alvéoles de la surface interne (4₂) texturée de l'enveloppe (3) est comprise entre 5 et 40 µm (microns).

9. Implant selon l'une quelconque des revendications 1 à 8, **caractérisé en ce que** la densité des alvéoles de la surface interne (4₂) texturée de l'enveloppe (3) est comprise entre 300 alvéoles/cm² et 1800 alvéoles/cm².

## Patentansprüche

1. Implantat (1) für die anatomische Rekonstruktion oder die Volumenvergrößerung eines Weichteils eines lebenden Körpers, wobei das Implantat aus einer Hülle (3) aus einem weichen und biokompatiblen Material besteht und mit einem Gel (2) gefüllt ist, wobei die mit dem Gel in Kontakt stehende Innenfläche (4₂) der Hülle (3) strukturiert ist, **dadurch gekennzeichnet, dass** die Innenfläche (4₂) offene Zellen mit mikroskopischen Abmessungen umfasst.

2. Implantat nach Anspruch 1, **dadurch gekennzeichnet, dass** die Hülle (3) aus Elastomer ist.

3. Implantat nach Anspruch 1 oder 2, **dadurch gekennzeichnet, dass** das Gel (2) ein Silikongel ist.

4. Implantat nach einem der Ansprüche 1 bis 3, **dadurch gekennzeichnet, dass** die Außenfläche (4₁) der Hülle (3) glatt ist.

5. Implantat nach einem der Ansprüche 1 bis 3, **dadurch gekennzeichnet, dass** die Außenfläche (4₁) der Hülle (3) strukturiert ist.

6. Implantat nach einem der Ansprüche 1 bis 5, **dadurch gekennzeichnet, dass** es sich bei dem Implantat (1) um ein rundes Brustimplantat handelt.

7. Implantat nach einem der Ansprüche 1 bis 5, **dadurch gekennzeichnet, dass** es sich bei dem Implantat (1) um ein Brustimplantat mit symmetrischer oder asymmetrischer anatomischer Form handelt.

8. Implantat nach einem der Ansprüche 1 bis 7, **dadurch gekennzeichnet, dass** die Tiefe der Zellen der strukturierten Innenfläche (4₂) der Hülle (3) zwischen 5 und 40 µm (Mikrometer) beträgt.

9. Implantat nach einem der Ansprüche 1 bis 8, **dadurch gekennzeichnet, dass** die Dichte der Zellen der strukturierten Innenfläche (4₂) der Hülle (3) zwischen 300 Zellen/cm² und 1800 Zellen/cm² beträgt.

## Claims

1. Implant (1) intended for anatomical reconstruction or for volumetric augmentation of a soft portion of a living body, which is formed by an envelope (3) made of a flexible and biocompatible material and filled with a gel (2), the internal surface (4₂) of the envelope (3) in contact with the gel being textured, **characterized in that** this internal surface (4₂) comprises open cells of microscopic dimensions.

2. Implant according to claim 1, **characterized in that** the envelope (3) is made of elastomer.

3. Implant according to any one of claims 1 or 2, **characterized in that** the gel (2) is a silicone gel.

4. Implant according to any one of claims 1 to 3, **characterized in that** the external surface (4₁) of the envelope (3) is smooth.

5. Implant according to any one of claims 1 to 3, **characterized in that** the external surface (4₁) of the envelope (3) is textured.

6. Implant according to any one of claims 1 to 5, **characterized in that** the implant (1) is a round breast implant.

7. Implant according to any one of claims 1 to 5, **characterized in that** the implant (1) is a breast implant with an anatomically symmetrical or asymmetrical shape.

8. Implant according to any one of claims 1 to 7, **characterized in that** the depth of the cells of the internal textured surface (4₂) of the envelope (3) is between 5 and 40 µm (microns).

9. Implant according to any one of claims 1 to 8, **characterized in that** the density of these cells of the internal textured surface (4₂) of the envelope (3) is between 300 cells/cm² and 1800 cells/cm².
